# EUROPÄISCHE PATENTSCHRIFT

(11) **EP 0 658 197 B1**
(45) Veröffentlichungstag und Bekanntmachung des Hinweises auf die Patenterteilung: **03.10.2007**
(21) Anmeldenummer: 93919151.6
(22) Anmeldetag: 24.08.1993
(51) Int. Cl.: C12N 15/00, A01K 67/027, C12N 15/90, C12N 15/13, C12N 5/10

(54) **GEZIELTE ERSETZTUNG EINES GENS OHNE ENDOGENE UND SELEKTIENBARE RESTSEQUENZEN**
TARGETED REPLACEMENT OF A GENE WITHOUT ENDOGENOUS AND SELECTABLE RESIDUAL SEQUENCES
REMPLACEMENT CIBLE D'UN GENE SANS SEQUENCES RESIDUELLES SELECTIONNABLES ET SANS ENDOGENES

(30) Priorität: 25.08.1992 DE 4228162
(43) Veröffentlichungstag der Anmeldung: 21.06.1995
(73) Patentinhaber: KÖLNER VEREIN ZUR FÖRDERUNG DER IMMUNOLOGIE, 50931 Köln (DE)
(72) Erfinder: RAJEWSKY, Klaus, D-50931 Köln (DE); ZOU, Yong-Rui, D-50937 Köln (DE)
(74) Vertreter: Helbing, Jörg
(86) Internationale Anmeldenummer: PCT/EP1993/002268
(87) Internationale Veröffentlichungsnummer: WO 1994/004667

(56) Entgegenhaltungen:
- WO-A-90/11354
- WO-A-91/09957
- WO-A-91/15579
- WO-A-91/19796
- WO-A-92/20808
- WO-A-93/01283
- CELL Bd. 73 , 18. Juni 1993 , CAMBRIDGE, NA US Seiten 1155 - 1164 GU, H. ET AL. 'Independent control of immunoglobulin switch recombination at invidual switch regions evidenced through Cre-loxP-mediated gene targeting'
- SCIENCE Bd. 259 , 12. Februar 1993 , LANCASTER, PA US Seiten 984 - 987 JUNG, S. ET AL. 'Shutdown of class switch recombination by deletion of a switch region control element' in der Anmeldung erwähnt
- PROCEEDINGS OF THE NATIONAL ACADEMY OF SCIENCES OF USA. Bd. 89, Nr. 14 , 1. Juli 1992 , WASHINGTON US Seiten 6232 - 6236 LAKSO, M. ET AL. 'Targeted oncogene activation by site-specific recombination in transgenic mice'
- PROCEEDINGS OF THE NATIONAL ACADEMY OF SCIENCES OF USA. Bd. 89, Nr. 15 , 1. August 1992 , WASHINGTON US Seiten 6861 - 6865 ORBAN, P.C. ET AL. 'Tissue- and site-specific DNA recombination in transgenic mice'
- MOLECULAR AND CELLULAR BIOLOGY Bd. 10, Nr. 9 , September 1990 Seiten 4466 - 4472 SCHULMAN, M.J. ET AL. 'Homologous recombination in hybridoma cells: dependence on time and fragment length'

### SEQUENZPROTOKOLL

(1) ALLGEMEINE INFORMATION:
   (i) ANMELDER:
      (A) NAME: Klaus Rajewsky
      (B) STRASSE: Bachemer Straße 95
      (C) ORT: Koeln
      (E) LAND: Deutschland
      (F) POSTLEITZAHL: 50931
   (ii) ANMELDETITEL: Verfahren zum Ersetzen homologer Genabschnitte aus Saeugern in der Keimbahn von nicht-menschlichen Saeugern
   (iii) ANZAHL DER SEQUENZEN: 4
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 21 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 1:
      CAGGATCCAA CTGTATCCAT C 21
(2) INFORMATION ZU SEQ ID NO: 2:
   (i) SEQUENZ CHARAKTERISTIKA:
      (A) LÄNGE: 21 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO : 2:
      GAGGTACCAA GGAAAGGGAG G 21
(2) INFORMATION ZU SEQ ID NO: 3:
   (i) SEQUENZ CHARAKTERISTIKA:
      (A) LÄNGE: 28 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 3:
      TTATCGATAC AGAGGCTCAA CCTACAAA 28
(2) INFORMATION ZU SEQ ID NO: 4:
   (i) SEQUENZ CHARAKTERISTIKA:
      (A) LÄNGE: 26 Basenpaare
      (B) ART: Nukleinsäure
      (C) STRANGFORM: nicht bekannt
      (D) TOPOLOGIE: nicht bekannt
   (ii) ART DES MOLEKÜLS : DNS (genomisch)
   (xi) SEQUENZBESCHREIBUNG: SEQ ID NO: 4:
      CCAAGCTTCG CTACTTTTGC ACCCTT 26

## Beschreibung

Die Erfindung betrifft ein Verfahren zur Erzeugung einer homozygoten transgenen Maus umfassend das Ersetzen eines Antikörpergens oder Antikörpergenabschnitts in der Keimbahn der Maus durch ein homologes Antikörpergen oder einen homologen Antikörpergenabschnitt eines Menschen.

Die Erfindung betrifft weiterhin die durch dieses Verfahren erhältliche transgene homozygote Maus sowie deren Verwendung zur Expression von Genprodukten.

Homologe Rekombination zwischen den in einem Chromosom vorhandenen DNA-Sequenzen und neuen, hinzugefügten, klonierten DNA-Sequenzen (im folgenden als "Gene-Targeting" bezeichnet) ermöglicht die Einfügung eines klonierten Gens in das Genom einer lebenden Zelle. Mit dieser Methode können bei Verwendung von embryonalen Keimzellen via Chimären Tiere erhalten werden, die homozygot für die gewünschte Mutation sind (M.R. Capecchi, Science 244, 1288 (1989)). In R. D. Camerini-Otero, R. Kucherlapati, The New Biologist, 2 (4), 334-341 (1990) ist die Verwendung des "Gene-Targeting" zur Inaktivierung eines Genes (gene disruption) und zur "Genkorrektur", d.h. Anfügen eines vorher nicht vorhandenen Genabschnittes, beschrieben.

Zur "Genkorrektur" sind dabei auch die in der WO 90/11354 und WO 91/19796 beschriebenen Insertionsverfahren zu zählen, bei denen ein gewünschter Genabschnitt durch homologe Rekombination in das Genom einer Zelle eingeführt wird. In der letztgenannten wird in einem zweiten Schritt das noch funktionsfähige endogene Gen entfernt, ein endogener Genabschnitt somit durch einen homologen Genabschnitt ersetzt. Darüber hinaus offenbart die WO 91/19796 noch ein virtuell einstufiges Verfahren, d.h. Cotransfektion, in dem der Resistenzmarker sich nicht in dem einzuführenden Genabschnitt befindet, sondern separat eingeführt wird. In den in dieser Druckschrift genannten Verfahrensbeispielen werden jedoch nur geringfügig variierte homologe Genabschnitte (max. 2x2 Basenvariationen) eingeführt, wobei sich die Frage stellt, inwieweit sich bei Abnahme der Homologie (bei der geringen Rekombinationsfrequenz des zweistufigen Verfahrens) noch ein selektierbares Rekombinationsereignis feststellen läßt.

Des weiteren ist in dieser Druckschrift nicht gezeigt, ob die durchgeführte Mutation der embryonalen Stammzellen in die Keimbahn übertragen wird.

Weiterhin regt K. Rajewsky (Science, 256, 483 (1992)) die Verwendung der homologen Rekombination zur Gensubstitution an, um z.B. die Funktion eines neu entdeckten Genes zu identifizieren.

In diesem Sinne bestand die Aufgabe der vorliegenden Erfindung darin, eine direkte, in einem Schritt erfolgreiche Methode zur Herstellung genmanipulierter Mäuse die homologe Antikörpergenabschnitte des Menschen enthalten, über homologe Rekombination zur Verfügung zu stellen.
Vom Anmelder wurde ein Verfahren gefunden, das es ermöglicht, in einem Schritt in der Keimbahn einer Maus gezielt einzelne Antikörpergenabschnitte durch Genabschnitte des Menschen zu ersetzen.

Die vorliegende Erfindung betrifft somit ein Verfahren zur Erzeugung einer homozygoten transgenen Maus durch homologe Rekombination, umfassend das Ersetzen eines Antikörpergens oder Antikörpergenabschnitts in der Keimbahn der Maus durch ein homologes Antikörpergen oder einen homologen Antikörpergenabschnitt eines Menschen, wobei
die embryonale Stammzelllinie der Maus mit einem selektierbar markierten Rekombinationsvehikel transfiziert wird, wobei das Rekombinationsvehikel ein Replacement-Vektor ist und den einzuführenden homologen Genabschitt oder das einzuführende homologe Gen, Sequenzen, die homolog zu den Sequenzen sind, welche den zu ersetzenden endogenen DNA-Abschnitt flankieren und ein Markergen umfasst, und in dem Rekombinationsereignis mit Hilfe des selektierbar markierten Rekombinationsvehikels das endogene Gen oder der endogene Genabschitt in einem Schritt durch das homologe Gen oder den homologen Genabschitt funktionell ersetzt wird;
stabil transfizierte Zellklone auf das Vorhandensein des Markergens selektiert werden; und
durch PCR und/oder Southern Blot gezielt selektiert werden.

Gemäß der vorliegenden Erfindung ist das selektierbar markierte Rekombinationsvehikel ein Replacement-Vektor und trägt den einzuführenden Genabschnitt bzw. das einzuführende Gen, Sequenzen, die homolog zu den Sequenzen sind, welche den zu ersetzenden endogenen DNA-Abschnitt flankieren und ein Markergen, insbesondere Neomycin oder Hygromycin, wobei Neomycin bevorzugt ist. Darüber hinaus kann das Rekombinationsvehikel noch virale Erkennungssequenzen (z.B. SV40), zusätzliche Sequenzen zur Verstärkung der Genexpression, Zielsequenzen für pro- und eukaryontische Rekombinationssysteme enthalten. Die letztgenannten Sequenzen, insbesondere die Cre-Erkennungssequenz LoxP oder die Flip-Erkennungssequenz Frt, können zum gezielten Entfernen der Markergene sowie von eventuell noch verbliebenen, nicht funktionellen Zielgenabschnitten verwendet werden. Es ist auf diese Weise möglich, im ersten Schritt den endogenen Genabschnitt durch einen homologen Genabschnitt eines anderen Säugers zu ersetzen und in einem zweiten Schritt die verbliebenen Reste mit Hilfe einer gezielt arbeiteten Rekombinase (H. Gu et al., Cell 73, 1155-1164 (1993)) zu entfernen. Bei diesem Verfahren wird der verbliebenen Rest gezielt entfernt und es kann, im Gegensatz zu der in der WO 91/19796 beschriebene "Hit and Run" Methode nur die gewünschte Rekombination stattfinden.

In einer bevorzugten Ausführungsform der vorliegenden Erfindung ist das selektierbar markierte Rekombinationsvehikel das Plasmid pTZ2-CkN (PA-) (DSM 7211).

Gemäß dem letztgenannten Verfahren werden die erfindungsgemäßen stabil transfizierten Zellklone in Mausblastozysten injiziert, diese Blastozysten in Leihmütter übertragen, die geborenen chimären Mäuse verpaart und deren Nachkommen auf das Vorhandensein der Mutation selektiert.

Auf diese Weise erhältliche homozygote transgene Mäuse sind ebenfalls Gegenstand der vorliegenden Erfindung.

Gegenstand der vorliegenden Erfindung ist weiterhin die Verwendung der gezielt mutierten homozygoten transgenen Mäuse zur Herstellung humanisierter monoklonaler Antikörper.

Die Erfindung wird weiter durch die folgenden Abbildungen erläutert:

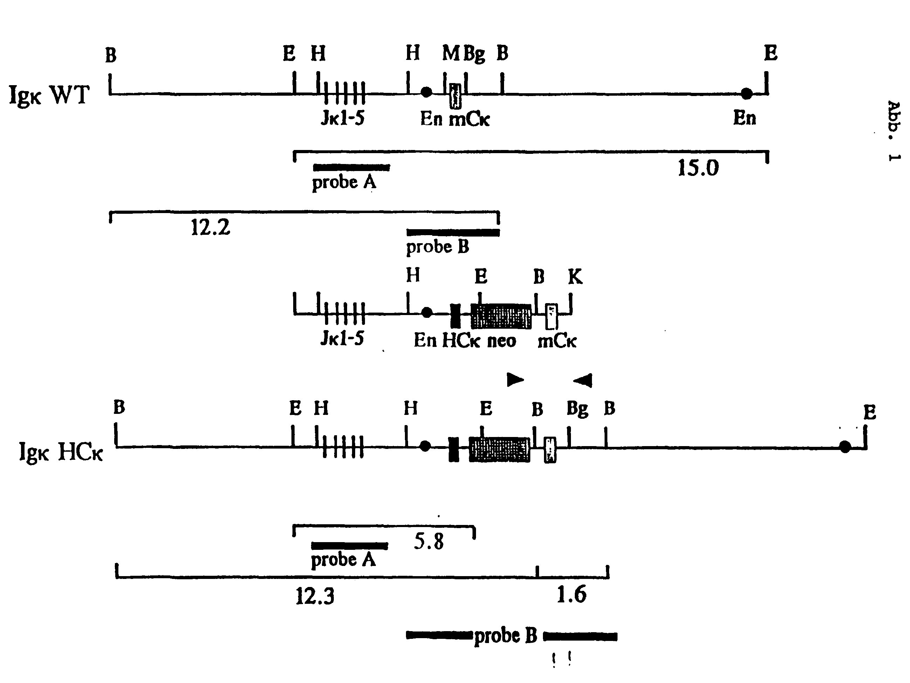

Abb. 1: Übersicht über die homologe Rekombination, die zum Austausch der murinen kappa-Kette (mCκ) durch die humane kappa-Kette (HCκ) führt. In der obersten Reihe ist der ursprüngliche Genabschnitt der konstanten Region der Maus-kappa-Kette, in der mittleren Reihe der zur gezielten Veränderung eingesetzte Vektor und in der untersten Reihe die neu entstandene Genstrüktur, in der der Genabschnitt mCκ (hell schraffierte Box) durch den Genabschnitt HCκ (schwarz gefüllte Box) in seiner Funktion ersetzt wurde, gezeigt. Darüber hinaus sind die Schnittstellen für Restriktionsenzyme, die entweder für die Konstruktion des Vektors oder für den Nachweis des veränderten Genabschnitts verwendet wurden (B, BamHI; E, Eco RI; H, Hpa I; M, Mst II; Bg, Bgl II; K, Kpn I) eingezeichnet. Eingezeichnet sind weiterhin DNA-Sonden, die für die Southern Blot Analysen verwendet wurden (Probe A und Probe B), sowie das für den Wildtyp und für das mutierte Gen erhaltene Fragment. In der untersten Reihe sind die Primer für die Polymerase-Kettenreaktion gezeigt, die für die Suche nach Zellen, in denen die gewünschte homologe Rekombination stattgefunden hat, verwendet wurden.

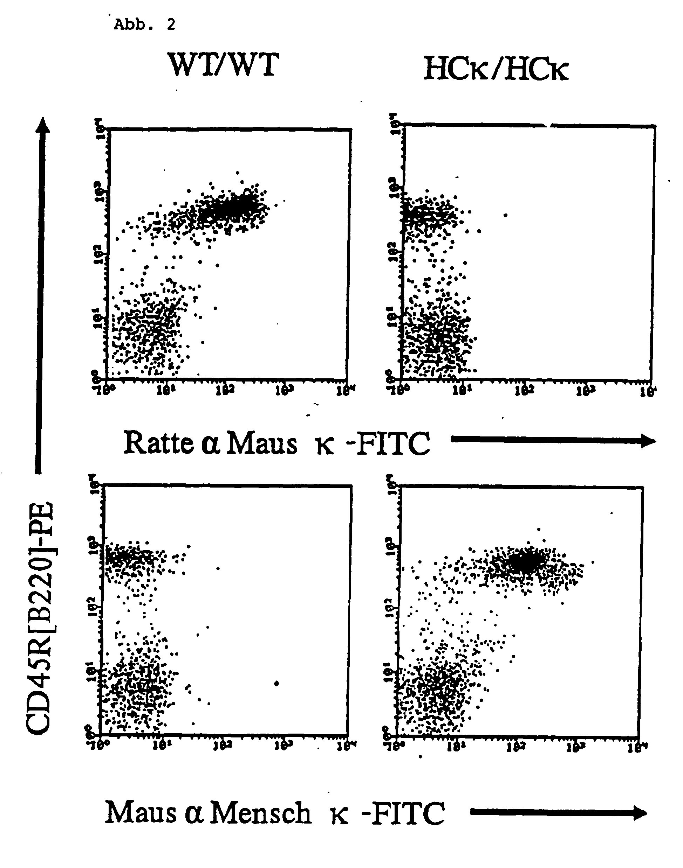

Abb. 2: Nachweis der Ausbringungen der kappa-Kette des Menschen anstelle der kappa-Kette der Maus in der Mausmutante (HCκ/HCκ). Milzzellen einer Wildtypmaus (WT/WT; linke Seite) und einer homozygoten Mausmutante (HCκHCκ; rechte Seite) wurden mit rot fluoreszenten Antikörpern gegen das B-Zellen-spezifische Antigen CD45R [B220], sowie mit grün fluoreszenten Antikörpern gegen entweder kappa-Ketten der Maus (oben) oder kappa-Ketten des Menschen (unten) gefärbt. Diese Zellen wurden in einem Durchflußcytometer analysiert. Das Ergebnis der Analyse ist in einem zweidimensionalen Punktediagramm dargestellt. Ein Punkt repräsentiert die Fluoreszenz einer Zelle. Ist eine Zelle nicht markiert, so erscheint sie in diesem Diagramm unten links. Eine Zelle, die nur rot fluoresziert, erscheint oben links, eine Zelle, die nur grün fluoreszent wäre, erschiene unten rechts und Zellen, die beide Fluoreszenzen tragen, oben rechts.

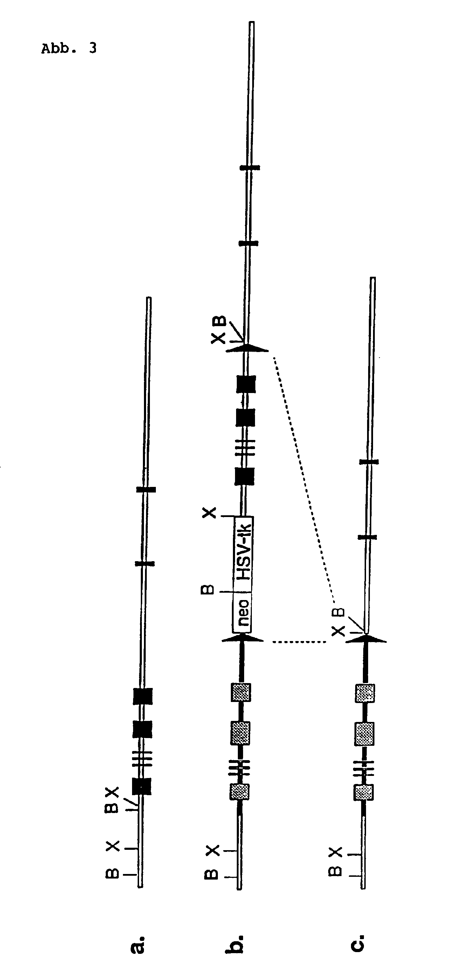

Abb. 3: Übersicht über die homologe Rekombination, die zum Austausch des Mausgens, das die konstante Region des IgG1 Gens kodiert, durch den entsprechenden humanen Genabschnitt unter Verwendung einer Rekombinase (CRE) in einem zweiten Reaktionsschritt führt.
   a: Dargestellt ist die Region des Maus IgGl-Gens vor der geplanten Veränderung. Die schwarzen Kästchen zeigen die Exons des Maus IgG1-Gens. Die Symbole B und X stehen für die Restriktionsenzymschnittstellen BamHI und XBaI.
   b: Gezeigt ist hier die Situation nach dem funktionellen Ersetzen der Maus IgG1-Region durch den entsprechenden Genabschnittes des Menschen (hellgraue Kästchen). Die schwarzen Dreiecke kennzeichnen die LoxP-Erkennungssequenzen, die die Selektionsmarker neo und HSV-tk sowie Reste des Maus IgG1-Gens flankieren.
   c: Hierin ist die Situation nach der Entfernung des von LoxP flankierten Bereiches durch die Rekombinase Cre gezeigt.

Das erfindungsgemäße Verfahren ermöglicht das Ersetzen von Antikörpergenen oder Genabschnitten in der Keimbahn von Mäusen durch ein homologes Antikörpergen oder einen homologen Antikörpergenabschnitt eines Menschen in einem Schritt. Via Chimären können dadurch Mäuse erhalten werden, die homozygot für die gewünschte Mutation sind und zur Expression von Genprodukten eines Menschen anstelle des endogenen Gens bzw. zum Austesten von Medikamenten und Therapiemodellen verwendet werden können.

Die vorliegende Erfindung wird durch die nachfolgenden Beispiele näher erläutert.

### Beispiele

### Beispiel I:

### Konstruktion des Gensubstitutionsvektors

1) Das Plasmid pTZ-HC_{κ} wurde konstruiert, indem ein 727 Basenpaar langes SphI-HhaI Fragment, das das Cx-Exon enthält, aus dem Vektor pC-2 (H.-G. Klobeck et al, Immunoglobulin genes of the κ light chain type from to human lymphoid cell lines are closely related. Nucleic Acids Research 12, 6995-7006 (1984)) zwischen die SphI und PstI Schnittstelle des Polylinkers des Plasmids pTZ-19(R) (Pharmacia LKB, Katalog Nr. 27-4986-01, Pharmacia LKB GmbH, Postfach 5480, Munzinger Str. 9, 7800 Freiburg) eingesetzt wurde.
2) Das Plasmid pTZ-HCκ-mCκ-5' wurde konstruiert, indem zwischen die HindIII und SphI Schnittstelle des Plasmids pTZ-HCx ein 1,2 kb HindIII-MstII Fragment vom Vektor pHBCx (S. Lewis, et al, Continuing Kappa-Gene Rearrangement in a cell line transformed by abelson murine leukemia virus; Cell 30, 807-816 (1982)), das das Intron-Enhancer-Element enthält, eingesetzt wurde.
3) Das Plasmid pTZ-5'HC&Neo wurde konstruiert, indem ein 1,1 kb XHOI-BamHI Fragment, das das Neomycin-Resistenzgen vom Plasmid pMCINeo (Stratagene, Katalog Nr. 213201, Stratagene GmbH, Postfach 105466, Im Weiher 12, 6900 Heidelberg) enthält, zwischen die SalI und BamHI Schnittstelle des Vektors pTZ-HCx-mCx5' eingesetzt wurde.
4) Das Plasmid pTZ-5'HRC&Neo wurde konstruiert, indem in die HindIII Schnittstelle das Plasmids pTZ-5'HC&Neo ein 2,8 kg langes HindIII Fragment vom Plasmid pHJκ (S. Lewis et al., loc. cit.), das die J 1-5 Elemente enthält, eingesetzt wurde.
5) Das Plasmid pTZ₂-CkN (PA⁻) (DSM 7211), das für die Gensubstitution verwendet wurde, enthält zusätzlich zwischen der BamHI und der KpnI Schnittstelle des Plasmids pTZ-5'-HRC&Neo ein 427 bp langes Fragment, das mit Hilfe der Polymerasekettenreaktion aus Mauskeimbahn DNA amplifiziert wurde. Um dieses Fragment aus der Mauskeimbahn DNA zu amplifizieren, wurden folgende Primer verwendet:
   5'-CAGGATCCAACTGTATCCATC hybridisiert 12 Basenpaare nach dem Beginn des Cκ Exons ; 5'-GAGGTACCAAGGAAAGGGAGG hybridisiert 137 Basenpaare nach dem Stopcodon TAG des Cκ Exon.

### Homologe Rekombination

Die Methode der homologen Rekombination mit Hilfe von Replacement-Vektoren, wie sie im Labor des Anmelders verwendet wird, ist mehrfach beschrieben worden (Kitamura et al, Nature 1991; Kühn et al, Science 1991; Kitamura et al, Cell 1992). Der für die homologe Rekombination verwendete Vektor enthält einen 4,5 kb großen Abschnitt genomischer DNA des murinen kappa-Genes (mC_{κ}). Dieser Teil enthält die Genabschnitte Jkl-5, den kappa-Intron-Enhancer und den 3' vom mC&Gen befindlichen nicht translatierten Bereich ohne die Polyadenylierungs-Stelle. Das humane kappa-Gen (HC_{κ}) und das für die Selektion notwendige Neomycin-Gen ohne eigene Polyadenylierungs-Stelle wurde zwischen die MstII und HpaI Restriktionsschnittstelle eingefügt und die zwischen MstII und HpaI befindliche Splicedonor-Stelle des mC_{κ} entfernt (Abb. 1). Da das Neomycin-Gen auf dem Vektor keine eigene Polyadenylierungs-Stelle trägt, wird für ein homologes Rekombinationsereignis selektioniert (ein Neomycin-resistenter Zellklon kann nur entstehen, wenn der Vektor vor einer Polyadenylierungs-Stelle im Genom integriert. Dieser Fall tritt z.B. ein, wenn der Vektor wie geplant homolog integriert). Der Vektor wurde linearisiert, durch Elektroporation in embryonale Stammzellen (ES) der Maus eingeführt und neomycin-resistente, das homologe Rekombinationsgereignis tragende ES-Zellen wurden durch Polymerase-Kettenreaktion (Abb. 1) und anschließenden Southern Blot identifiziert (Abb. 1). In einem Experiment wurden 2x10⁷ ES-Zellen transfiziert. Eine aus 480 neomycin-resistenten ES-Zellen trug die geplante Mutation (Abb. 1).

Um die Ausprägung der geplanten Veränderung zu untersuchen, wurden mit Hilfe eines ES-Zellklones chimäre Mäuse hergestellt. Diese chimären Mäuse wurden mit C57BL/6 Mäusen verpaart. In der nächsten Generation wurden Mäuse, die die Mutation tragen, verpaart. In der dann folgenden Generation werden Mäuse geboren, die zwei unveränderte Maus Kappa Loci tragen (WT) (25 %), die zwei mutierte Loci tragen (HC_{κ} ) (25 %) sowie Mäuse, die je einen unveränderten Maus Kappa Locus und einen mutierten Locus tragen (50 %). Gezeigt wird im folgenden der Vergleich zwischen einer Wildtyp-Maus (WT/WT) und einer Maus homozygot für die Mutation (HC_{κ}/HC_{κ}).

Um zu zeigen, daß in der homozygoten Mausmutante das HC&Gen für die Antikörperbildung verwendet wird, wurde die Konzentration von Antikörpern, die die konstante Region des humanen Kappa Gens tragen, im Serum der Mäuse durch Serologie bestimmt. In einer Wildtyp-Maus sind Antikörper dieser Art nicht nachweisbar. Die Nachweisgrenze liegt bei 250 ng/ml. In Seren von Mäusen, die ein HCκ tragen, wird eine Konzentration von 178 µg/ml, in Mäusen, die homozygot für diese Mutation sind, eine Konzentration von 2860 µg/ml gefunden. Zum Vergleich: Ein normales Serum des Menschen enthält ca. 15000 µg/ml.

Der direkte Beweis, daß das HCκ Gen das mCκ Gen funktionell ersetzt, wird durch eine Analyse der Antikörper-produzierenden Zellen (B-Lymphozyten) in der Maus erbracht. Ruhende B-Lymphozyten tragen auf ihrer Oberfläche die Antikörper, die sie in ihrem Genom nach erfolgter Genumlagerung ausprägen. Diese Antikörper lassen sich durch markierte Antikörper, spezifisch für leichte Ketten, nachweisen. Ein Experiment, in dem Antikörper spezifisch entweder für die Kappa-Kette der Maus oder für die kappa-Kette des Menschen verwendet wurden, ist in Abb. 2 gezeigt. Um B-Lymphozyten zu markieren, wurden die Zellen mit einem rot fluoreszierenden Antikörper, der alle B-Zellen erkennt (anti CD45R[B220]) markiert. Gleichzeitig wurden die Zellen mit einem grün fluoreszierenden Antikörper, spezifisch für die konstante Region der kappa-leichten Kette markiert. Man erkennt, daß in der Mausmutante B-Lymphozyten Antikörper mit der humanen kappa-Kette ausprägen, die kappa-Kette der Maus wird nicht mehr verwendet.

### Beispiel II:

### Konstruktion des Gensubstitutionsvektors

1) Zur Kontruktion des Vektors pG1 wurde zunächst das die homologe Region des kurzen Armes des IgG1 kodierende Plasmid pG1A, welches das Mäuse γ₁-Gen und seine flankierenden Regionen enthält mittels PCR isoliert. Hierzu wurde eine Mischung von jeweils 10 pmol der folgenden Primer TTATCGATACAGAGGCTCAACCTACAAA und CCAAGCTTCGCTACTTTTGCACCCTT sowie 10 ng der Plasmid DNA als Matrize 25 Cyclen - 94°C (1 min), 69°C (1,5 min) und 74°C (2 min) unterworfen. Zur Herstellung des p5'HROGNT-Vektors wurde die so isolierte homologe Region in eine neo^{r}-tk Kassette (H. Gu et al., Cell 73,, 1155-1164 (1993)), die einer Frt-Stelle (O'Gorman et al., Science 251, 1351 (1991) am 5'Ende aufwies kloniert. Der p5'HROGNT-Vektor wurde partiell mit BamHI und nachfolgend mit XhoI aufgeschlossen. Ein 1,2 kb-BamH-XhoI-Fragment, das ein neo^{r}-Gen, welches an seinem 5'Ende eine loxP-Stelle aufweist, enthält, wurde von dem Plasmid pGH1 (H. Gu et al., Cell 73, 1155-1164 (1993)) isoliert und zur Herstellung des Vektors pG1 in den aufgeschlossenen p5'HROGNT-Vektor kloniert.
2) Zur Herstellung des Vektors pG2 wurde das humane γ₁-Gen, das die sekretorische Form des humanen IgGl kodiert, in den pG1-Vektor subkloniert. Hierzu wurde ein 2,1 kb HindIII-PvuII-Fragment, welches das humane γ₁-Gen enthielt, aus dem Plasmid pTJ1B (A. Kudo et al., Gene 33, 181 (1985)) isoliert. Ein weiteres Fragment, welches das neo^{r}-Gen und ein Teil des tk-Gens enthielt wurde aus dem pG1-Plasmid durch XhoI-Aufschluß, nachfolgendem T₄-Polymerase Auffüllen und Aufschluß mit BgIII isoliert. Weiterhin wurde der pG1-Vektor teilweise mit HindIII und nachfolgend mit XhoI-BgIII aufgeschlossen. Zur Konstruktion des pG2-Vektors wurden diese drei Fragmente ligasiert.
3) Zur Konstruktion des Vektors pG3 wurde eine loxP Stelle vor die Mäuse γ1 Membrane Exons subkloniert. Hierzu wurde das Plasmid pTZ-3' γ₁ (4.3) und das Plasmid pGEM30 (H. Gu et al., Cell 73, 1155-1164 (1993)) partiell mit EcoRI und nachflogend mit SalI aufgeschlossen und miteinander ligasiert. Das Plasmid pTZ-3' γ₁ (4.3), welches zwei Membranen Exons des Mäuse γ₁-Gens enthält, wurde aus einem 1,5 kb SacI-EcoRI-Fragment des Plasmids pG1A und einem 2,8 kb EcoRI-Fragment des Bakteriophagen ch γ₁₋₃ (A. Schimizu et al., Cell 28, 499 (1982)) hergestellt.
4) Zur Konstruktion der gesamten 3' homologen Region des Gensubstitutionsvektors wurde der Vektor pGH2, der ein genomisches 6,3-XbaI-EcoRI genomisches DNA-Fragment einschließlich des Maus γ₁-Gens in der sekretorischen und membranen Form enthält, mit SacI aufgeschlossen, mit T₄ Polymerase abgefüllt und dann mit BglII aufgeschlossen. Der pG3 Vektor wurde mit XhoI geschnitten, mit T₄ Polymerase aufgefüllt und dann mit BglII und SphI geschnitten. Zur Konstruktion des Vektors pG4 wurden die beiden so erhaltenen Fragmente ligasiert.
5) Zur Konstruktion des Gensubstitutionsvektors pG5 wurde der Vektor pG4 mit ClaI und XhoI, der Vektor pG2 mit ClaI und SalI geschnitten und dann miteinander ligasiert.

### Homologe Rekombination

Zur homolgen Rekombination wurde der Vektor pG5 durch ClaI Aufschluß linearisiert und wie in Beispiel 1 beschrieben durch Elektroporation in embryonale Stammzellen der Maus eingeführt.

Nachfolgend wurde in einem weiteren Schritt die Rekombinase transient in den embryonalen Stammzellen ausgeprägt (H. Gu et al., Cell 73, 1155-1164 (1993)). Die Rekombinase entfernt dann in hoher Effizient den von der Erkennungssequenz markierten Bereich (Abbildung 3). Nachfolgend wurde analog Beispiel I via chimären Mäusen eine Mausmutante erzeugt, die homozygot für die Mutation ist.

Der Nachweiß, daß das Mausgen, das die konstante Region des IgGl-Gens kodiert durch den entsprechenden Bereich des humanen Gens ersetzt wurde, wurde durch Nachweiß von humanen IgGl in Kulturen von B-Zellen der entsprechenden chimären Maus bestätigt: In Kulturen von drei Mäusen von Konzentrationen von je 2 µg/ml humanes IgG1 gemessen, während in den Kontrollkulturen die Werte unter der Nachweisgrenze von 0,1 µg/ml blieben.

Diese Beispiele zeigen, daß es durch homologe Rekombination möglich ist, homologe Gene oder Genabschnitte aus Säugern (hier: Mensch) in der Keimbahn von nicht-menschlichen Säugern (hier: Maus) in einem Schritt funktionell zu ersetzen. Desweiteren kann durch Verpaaren einer Maus aus Beispiel I mit einer Maus aus Beispiel II eine Mausmutante erhalten werden, bei der sowohl das &-Gen als auch das γ₁-Gen vom Menschen stammt. Solch eine Mausmutante ist daher zur Herstellung von humanisierten monoklonaler Antikörper geeignet.

Das zur Gensubstitution verwendete Plasmid (pTZ₂-CkN (PA⁻) wurde bei der Deutschen Sammlung von Mikroorganismen und Zellkulturen GmbH, Maschorder Weg 1b, W-3300 Braunschweig, als E. coli Stamm DSM 7211 hinterlegt.

## Patentansprüche

1. Verfahren zur Erzeugung einer homozygoten transgenen Maus durch homologe Rekombination, umfassend das Ersetzen eines Antikörpergens oder Antikörpergenabschnitts in der Keimbahn der Maus durch ein homologes Antikörpergen oder einen homologen Antikörpergenabschnitt eines Menschen, wobei
(i) die embryonale Stammzelllinie der Maus mit einem selektierbar markierten Rekombinationsvehikel transfiziert wird, wobei das Rekombinationsvehikel ein Replacement-Vektor ist und den einzuführenden homologen Genabschnitt oder das einzuführende homologe Gen, Sequenzen, die homolog zu den Sequenzen sind, welche den zu ersetzenden endogenen DNA-Abschnitt flankieren und ein Markergen umfasst, und in dem Rekombinationsereignis mit Hilfe des selektierbar markierten Rekombinationsvehikels das endogene Gen oder der endogene Genabschnitt in einem Schritt durch das homologe Gen oder den homologen Genabschnitt funktionell ersetzt wird;
(ii) stabil transfizierte Zellklone auf das Vorhandensein des MarkerGens selektiert werden; und
(iii) durch PCR und/oder Southern Blot gezielt selektiert werden.

2. Verfahren nach Anspruch 1, wobei
(i) in dem selektierbar markierten Rekombinationsvehikel das Markergen ausgewählt ist aus Neomycin und Hygromycin und insbesondere Neomycin ist und/oder
(ii) das Rekombinationsvehikel noch zusätzliche funktionale Gensequenzen, insbesondere virale Erkennungssequenzen, Sequenzen zur Verstärkung der Genexpression und Zielsequenzen für pro- und eukaryontische Rekombinationssysteme insbesondere die Cre-Erkennungssequenz LoxP und Flip-Erkennungssequenz Frt, enthält .

3. Verfahren nach Anspruch 2, **dadurch gekennzeichnet, daß** das Markergen Neomycin ist.

4. Verfahren nach einem oder mehreren der Ansprüche 1 bis 3, **dadurch gekennzeichnet, daß** das selektierbar markierte Rekombinationsvehikel pTZ2-CkN (PA-) (DSM 7211) ist.

5. Verfahren nach Anspruch 2, **dadurch gekennzeichnet, daß** mittels der Zielsequenzen in einem zweiten Reaktionsschritt Markergene und noch vorhandene Zielgenabschnitte entfernt werden.

6. Homozygote transgene Maus, die durch ein Verfahren nach einem oder mehreren der Ansprüche 1 bis 5 erhältlich ist.

7. Verwendung der homozygoten Maus nach Anspruch 6 zur Expression von humanisierten monoklonalen Antikörpern.

## Claims

1. A method of producing a homozygous transgenic mouse by homologous recombination, comprising the step of replacing an antibody gene or antibody gene segment in the germ line of the mouse by a homologous antibody gene or a homologous antibody gene segment of a human, wherein:
(i) the embryonic stem cell line of the mouse is transfected with a recombination vehicle comprising a selectable marker, wherein said recombination vehicle is a replacement vector comprising the homologous gene segment to be introduced or the homologous gene to be introduced, sequences being homologous to the sequences flanking the endogenous DNA segment to be replaced, and a marker gene, and in the recombination event, the endogenous gene or gene segment is functionally replaced by said homologous gene or said homologous gene segment in one step by means of said recombination vehicle comprising a selectable marker;
(ii) stably transfected cell clones are selected for the presence of the marker gene; and
(iii) purposefully selected by PCR and/or Southern blotting.

2. The method according to claim 1, wherein:
(i) in said recombination vehicle comprising a selectable marker, said marker gene is selected from neomycin and hygromycin, especially neomycin; and/or
(ii) said recombination vehicle comprises additional functional gene sequences, especially viral recognition sequences, sequences for enhancing gene expression, and target sequences for prokaryotic and eukaryotic recombination systems, especially the Cre recognition sequence LoxP and flip recognition sequence Frt.

3. The method according to claim 2, **characterized in that** said marker gene is neomycin.

4. The method according to one or more of claims 1 to 3, **characterized in that** said recombination vehicle comprising a selectable marker is pTZ2-CkN (PA-) (DSM 7211).

5. The method according to claim 2, **characterized in that** marker genes and any target gene segments still present are removed in a second reaction step by means of target sequences.

6. A homozygous transgenic mouse obtainable by a method according to one or more of claims 1 to 5.

7. Use of the homozygous mouse according to claim 6 for the expression of humanized monoclonal antibodies.

## Revendications

1. Procédé de génération d'une souris transgénique homozygote par recombinaison homologue, comportant le remplacement d'un gène d'anticorps ou d'un segment de gène d'anticorps dans la ligne germinale de la souris par un gène d'anticorps homologue ou un segment de gène d'anticorps homologue d'un homme, dans lequel
(i) la lignée cellulaire souche embryonnaire de la souris est transfectée avec un véhicule de recombinaison marqué de façon pouvant être sélectionnée, où le véhicule de recombinaison est un vecteur de remplacement et comprend le segment de gène homologue à introduire ou le gène homologue à introduire, des séquences qui sont homologues aux séquences qui flanquent le segment d'ADN endogène à remplacer et un gène marqueur, et dans l'événement de recombinaison, à l'aide du véhicule de recombinaison marqué de façon pouvant être sélectionnée, le gène endogène ou le segment de gène endogène est remplacé de façon fonctionnelle en une étape par le gène homologue ou le segment de gène homologue ;
(ii) des clones cellulaires transfectés de façon stable sont sélectionnés sur la présence du gène marqueur ; et
(iii) sont sélectionnés de façon ciblée par PCR et/ou Southern blot.

2. Procédé selon la revendication 1, dans lequel
(i) dans le véhicule de recombinaison marqué de façon pouvant être sélectionnée, on sélectionne le gène marqueur parmi la néomycine et l'hygromycine, et il est en particulier la néomycine et/ou
(ii) le véhicule de recombinaison contient encore des séquences de gènes fonctionnelles supplémentaires, en particulier des séquences de reconnaissance virales, des séquences permettant de renforcer l'expression d'un gène et des séquences cibles pour des systèmes de recombinaison procaryotes et eucaryotes, en particulier la séquence de reconnaissance de Cre LoxP et la séquence de reconnaissance de Flip Frt.

3. Procédé selon la revendication 2, **caractérisé en ce que** le gène marqueur est la néomycine.

4. Procédé selon une ou plusieurs des revendications 1 à 3, **caractérisé en ce que** le véhicule de recombinaison marqué de façon pouvant être sélectionnée estpTZ2-CkN (PA-) (DSM 7211).

5. Procédé selon la revendication 2, **caractérisé en ce qu'**au moyen des séquences cibles, dans une seconde étape de réaction, on retire les gènes marqueurs et les segments de gène cible encore présents.

6. Souris transgénique homozygote qui peut être obtenue par un procédé selon une ou plusieurs des revendications 1 à 5.

7. Utilisation de la souris homozygote selon la revendication 6 pour l'expression d'anticorps monoclonaux humanisés
